# EUROPEAN PATENT SPECIFICATION

(11) **EP 1 768 783 B1**
(45) Date of publication and mention of the grant of the patent: **19.03.2008**
(21) Application number: 05745679.0
(22) Date of filing: 26.05.2005
(51) Int. Cl.: B01L 3/00, G01N 33/48

(54) **CONTROLLED FLOW ASSAY DEVICE AND METHOD**
ASSAYVORRICHTUNG UND VERFAHREN MIT GESTEUERTEM FLUSS
DISPOSITIF ET PROCEDE DE DOSAGE A ECOULEMENT REGULE

(30) Priority: 02.06.2004 SE 0401424
(43) Date of publication of application: 04.04.2007
(73) Proprietor: Amic AB, 751 83 Uppsala (SE)
(72) Inventor: ÖHMAN, Per, Ove, S-755 91 Uppsala (SE); MENDEL-HARTVIG, Ib, S-756 55 Uppsala (SE)
(74) Representative: Holmberg, Martin Tor
(86) International application number: PCT/SE2005/000787
(87) International publication number: WO 2005/118139

(56) References cited:
- EP-A1- 1 371 984
- WO-A1-92/01226
- WO-A1-03/103835
- US-B1- 6 368 871

## Description

The present invention relates to the field of assay devices or assay systems including components thereof, for use in the detection of one or more analytes in a sample, as well as method for the use of said device or component, and methods for detection of an analyte, using said device. The invention in particular relates to such devices and such methods where the flow of liquids is controlled.

### Background

Analytical and diagnostic determinations are frequently performed on liquid samples, comprising in addition to the analyte of interest, also countless other components, in solution and/or in particulate form, which often interfere with the handling of the sample and may influence the quantitative or qualitative determination of the analyte.

For example, numerous clinical diagnostic methods are based on the detection of an analyte in a biological sample. Frequently, such detection is achieved in a disposable assay device, allowing rapid and simple diagnosis. One important application is the wide field of immunology, where analytes are detected with the aid of specific antibodies, capable of binding to the analytes and forming detectable complexes, usually with the aid of ligands aiding the detection.

When performing a test using a biological sample from a patient, in particular a blood sample, many factors need to be considered. Whole blood is prone to clotting, reducing or preventing the desired flow of the sample in the assay device. The red blood cells, even in the absence of clotting, may inhibit or retard flow. Further, red blood cells may inhibit binding between specific binding pair members. Red blood cells also have enzymatic activity, which, depending on the assay employed, may interfere with the signal produced.

Unfortunately, red blood cells present in whole blood also scatter and absorb light thus interfering with assay methodologies which measure either reflected or transmitted light. Also other cells may interfere with particular determinations; for example, cholesterol determinations can be effected by cholesterol present in cell membranes.

Further, the red cell fraction takes up a considerable volume of the sample, in some cases as much as half the volume. Importantly, this fraction, also called hematocrit, may vary between different individuals and even in the same individual, between different measurements. This in turn may influence the accuracy and/or the repeatability of the determinations.

Consequently many assays involve a step of separating the red blood cells from the plasma, whereupon the assay is carried out on plasma or serum. When the separation is performed before clotting, plasma is obtained. When clotting has occurred before separation, serum is obtained.

The red blood cells can be separated from plasma through centrifugation, which however requires relatively large volume of sample, and the use of a centrifuge. This is also time consuming and constitutes an additional step of handling the sample, which increases cost and complexity, and which should be avoided in particular when potentially contagious blood-borne pathogens are involved. Further, the risk of the sample being contaminated by the individuals handling it, cross-contaminated by parallel sample or mixed up with other samples is increased.

What is said above regarding whole blood samples and red blood cells applies also, with necessary adaptations, to other biological samples, where cells, cell debris, fibres, or other unwanted particles etc., may interfere with the determination and should therefore preferably be separated before or during the reaction or determination leading to the detection of the analyte.

The most common type of disposable assay device consists of a zone or area for receiving the sample, a reaction zone, and optionally a transport or incubation zone connecting the receiving and reaction zone, respectively. These assay devices are known as chromatography assay devices or simply referred to as strip tests. They employ a porous material defining a path for fluid flow capable of supporting capillary flow, e.g. a filter material. The sample-receiving zone frequently consists of a more porous material, capable of absorbing the sample, and, when the separation of blood cells is desired, effective to trap the red blood cells. Examples of such materials are fibrous materials, such as paper, fleece, gel or tissues, comprised e.g. of cellulose, wool, glass fibre, asbestos, synthetic fibres, polymers or mixtures of the same. The transport or incubation zone commonly consists of the same or similar materials, often with another porosity than the sample-receiving zone. Likewise, the reaction zone, which may be integrated with the incubation zone, or constituting the most distal part thereof, commonly consists of similar, absorbing fibrous materials, or any of the above listed materials.

In a conventional assay device or strip test, the porous material (-s) is (are) assembled on a carrier, such as a strip of thermoplastic material, paper, cardboard or the like. Further, a cover can be provided, said cover having at least one aperture for receiving the sample, and an aperture or transparent area for reading the result of the assay.

Nitrocellulose materials are also frequently used as the matrix constituting the transport or reaction zone, connecting the receiving zone and the reaction zone. A significant disadvantage with nitrocellulose is its high non-specific binding of proteins and other bio-molecules. Present test strips however often handle a surplus of sample, reducing the influence of this binding. It is however desirable to minimise the sample volume, in line with the tendency to miniaturize the entire test, including minimising the amounts of reagents without compromising accuracy and reliability.

### Prior art

EP 1 371 984 discloses a chromatographic assay device and method for detecting the presence of an analyte in a sample of whole blood, utilizing a red blood cell separating agent to aggregate red blood cells and permit plasma or serum to flow by capillary action. The carrier material is exemplified as a paper (fibrous), or membranes of cellulose, fibreglass, cloth, both naturally occurring and synthetic, as well as porous gels.

Although frequently used and well known in the art, the above carrier materials are associated with many drawbacks. The structure of the materials will always vary between different batches, and also within the material, due to the random distribution of the fibres e.g. in a fibrous material, or cavities e.g. in a gel-like material. Similarly, the chemical properties of the material, e.g. the distribution of chemicals added to the material, will inevitable vary for the same reasons as above.

WO 03/103835 discloses micro fluidic systems comprising a substrate, and, provided on said substrate, at least one flow path interconnecting with functional means in which liquid samples can be subjected to different desired procedures, said flow path comprising a plurality of micro posts protruding form said substrate.

The objective of the present invention is to further develop the micro fluidic systems disclosed in WO 03/103835, and in particular to make available means for controlling or regulating the flow, including enhancing or attenuating the flow of liquid samples, reagents or other components on said substrate.

### Summary of the invention

The present invention makes available a device for the detection of an analyte in a liquid sample, or a component of such device, said device comprising a flow path with at least one zone for receiving the sample, and a transport or incubation zone, said zones connected by or comprising an area having projections substantially vertical to its surface, wherein said device further comprises a sink with a capacity of receiving and/or absorbing said liquid sample and supporting or controlling the flow rate of said sample through said transport or incubation zone, said sink comprising an area having projections substantially vertical to its surface, and said sink being adapted to respond to external influence regulating its capacity to receive said liquid sample.

The invention also encompasses embodiments of said device and method, as set forth in the description and claims.

### Short description of the drawings

The invention will be described in closer detail in the following description, examples, and attached drawings, in which
Fig. 1 schematically shows a cross section of a device according to the invention, where a means for heating causes evaporation of liquid at one end, thus driving the flow along the flow path of the device;
Fig. 2 schematically shows a top view of a device according to the invention, where the heating is performed in zones, starting from the zone most distal to the point where the sample is added;
Fig. 3 schematically shows an embodiment of the invention where two parallel determinations can be performed, the flow rate and thereby incubation time individually adjusted in the two flow paths;
Fig. 4 schematically shows another embodiment where sample, reagents and buffers can be serially added and transported along a flow path in a controlled manner; and
Fig. 5 shows different embodiments, illustrating how the transition from one flow path to another can be arranged: A) using a difference in the geometry of the micro posts; B) using a difference in the height and spacing of the micro posts; and C) using the design of the connection between the flow paths.

### Description

### Definitions

Before the present device and method is described, it is to be understood that this invention is not limited to the particular configurations, method steps, and materials disclosed herein as such configurations, steps and materials may vary somewhat. It is also to be understood that the terminology employed herein is used for the purpose of describing particular embodiments only and is not intended to be limiting since the scope of the present invention will be limited only by the appended claims and equivalents thereof.

It must also be noted that, as used in this specification and the appended claims, the singular forms "a", "an", and "the" include plural referents unless the context clearly dictates otherwise. Thus, for example, reference to a reaction mixture containing "a monoclonal antibody" includes a mixture of two or more antibodies.

The term "about" when used in the context of numeric values denotes an interval of accuracy, familiar and acceptable to a person skilled in the art. Said interval can be ± 10 % or preferably ± 5 %.

In describing and claiming the present invention, the following terminology will be used in accordance with the definitions set out herein.

The term "sample" here means a volume of a liquid, solution or suspension, intended to be subjected to qualitative or quantitative determination of any of its properties, such as the presence or absence of a component, the concentration of a component, etc. The sample may be a sample taken from an organism, such as a mammal, preferably a human; or from the biosphere, such as a water sample, or an effluent; or from an technical, chemical or biological process, such as a process of manufacturing, e.g. the production of medicaments, food, feed, or the purification of drinking water or the treatment of waste effluents. The sample may be subjected to qualitative or quantitative determination as such, or after suitable pre-treatment, such as homogenisation, sonication, filtering, sedimentation, centrifugation, heat-treatment etc.

Typical samples in the context of the present invention are body fluids such as blood, plasma, serum, lymph, urine, saliva, semen, gastric fluid, sputum, tears etc.; environmental fluids such as surface water, ground water, sludge etc.; and process fluids such as milk, whey, broth, nutrient solutions, cell culture medium, etc. The present invention is applicable to all samples, but preferably to samples of body fluids, and most preferably to whole blood samples.

The determination based on lateral flow of a sample and the interaction of components present in the sample with reagents present in the device and detection of such interaction, either qualitatively or quantitatively, may be for any purpose, such as diagnostic, environmental, quality control, regulatory, forensic or research purposes. Such tests are often referred to as chromatography assays, or lateral flow assays, as in e.g. immunochromatography assays.

Examples of diagnostic determinations include, but are not limited to, the determination of analytes, also called markers, specific for different disorders, e.g. chronic metabolic disorders, such as blood glucose, blood ketones, urine glucose (diabetes), blood cholesterol (atherosclerosis, obesitas, etc); markers of other specific diseases, e.g. acute diseases, such as coronary infarct markers (e.g. troponin-T), markers of thyroid function (e.g. determination of thyroid stimulating hormone (TSH)), markers of viral infections (the use of lateral flow immunoassays for the detection of specific viral antibodies); etc.

Another important field of diagnostic determinations relate to pregnancy and fertility, e.g. pregnancy tests (determination of i.a. human chorionic gonadotropin (hCG)), ovulation tests (determination of i.a. luteneizing hormone (LH)), fertility tests (determination of i.a. follicle-stimulating hormone (FSH)) etc.

Yet another important field is that of drug tests, for easy and rapid detection of drugs and drug metabolites indicating drug abuse; such as the determination of specific drugs and drug metabolites (e.g. THC) in urine samples etc.

The term "analyte" is used as a synonym of the term "marker" and intended to encompass any substance that is measured quantitatively or qualitatively.

The terms "zone", "area" and "site" are used in the context of this description, examples and claims to define parts of the flow path on a substrate, either in prior art devices or in a device according to the invention.

The term "reaction" is used to define any reaction, which takes place between components of a sample and at least one reagent or reagents on or in said substrate, or between two or more components present in said sample. The term "reaction" is in particular used to define the reaction, taking place between an analyte and a reagent as part of the qualitative or quantitative determination of said analyte.

The term "substrate" here means the carrier or matrix to which a sample is added, and on or in which the determination is performed, or where the reaction between analyte and reagent takes place.

The term "chemical functionality" comprises any chemical compound or moiety necessary for conducting or facilitating the assay. One group of chemical compounds, with particular relevance in the present invention, are compounds or components exhibiting specific affinity to, or capability of binding or interacting with, one or more components in the sample. Red blood cell separating agents constitute an illustrative example. Such agents may be any substance capable of aggregating or binding red blood cells.

The term "biological functionality" comprises all biological interactions between a component in a sample and a reagent on or in the substrate, such as catalysis, binding, internalisation, activation, or other biospecific interaction. Suitable reagents include, but are not limited to, antibodies, antibody fragments and derivates, single chain antibodies, lectines, DNA, aptamers, etc., including other polymers or molecules with binding capacity. Such reagents can be identified by a person skilled in the art, following the choice of the component to be separated, using standard experimentation, e.g. screening methods and chemical libraries.

The term "physical functionality" here comprises functionalities involved in reactions and interactions other than those that are mainly chemical or biological. Examples include diameter, height, shape, cross section, surface topography and surface patterns, the number of projections per unit area, wetting behavior of the surface of said projections, or a combination thereof, and/or other functionalities influencing the flow, retention, adhesion or rejection of components of the sample.

The distinctions between chemical, biological and physical interactions are not always clear, and it is possible that an interaction - such as an interaction between a component in a sample and a reagent on the substrate - involves chemical, biological as well as physical elements.

The terms "hydrophilic" and "hydrophobic", as in hydrophilic or hydrophobic compounds, hydrophilic or hydrophobic interactions etc., have the meaning generally understood by a person skilled in the art, and corresponding to that used in generally recognised textbooks.

### Description of preferred embodiments

The present invention makes available a device for handling liquid samples, said device comprising a flow path with at least one zone for receiving the sample, and a transport or incubation zone, said zones connected by or comprising an area having projections substantially vertical to its surface, wherein said device further comprises a sink with a capacity of receiving said liquid sample and supporting or controlling the flow rate of said sample through said transport or incubation zone, said sink comprising an area having projections substantially vertical to its surface, and said sink being adapted to respond to external influence regulating its capacity to receive said liquid sample.

The device according to the invention can also comprise two or more flow paths, each connected to a sink, said device being adapted for performing multiple analyses on one sample. In this case, each flow path comprises a reaction zone, and individual reagents, such as conjugates, buffers etc are added to or stored in each flow path or reaction zone. A device according to this embodiment is advantageous, as it makes it possible to perform multiple analyses in parallel or substantially in parallel, starting from one sample, added to one device.

According to one embodiment, multiple reagents, buffers, etc are serially added to one flow path. This means that each one of sample, reagent, buffer etc will ravel along the flow path and pass the reaction zone in a predetermined order. Using the sink, and in particular a heated sink, the flow speed of each component can be controlled. This makes it possible to perform an e.g. analysis involving a slow pre-treatment, an incubation of a predetermined length, followed by a rapid rinse etc, only to mention an example.

According to the invention, the external influence regulating the capacity of said sink to receive said liquid sample is chosen among heating, cooling, irradiation with visible light, infrared irradiation, vibration, and the application of an electric current.

According to an embodiment of the invention, the sink is divided into sub sections, suitable for being serially subjected to said external influence. This is advantageous e.g. in instances where the sample tends to coagulate, denaturate or simply to dry in the sink during the heating. By serially heating sections of the sink, starting from the most distal one, it is possible to retain the aspirating or absorbing capacity of the sink.

One embodiment of the invention concerns the provision of means or design measures creating a preferred direction of flow within the device. Such means or measures can comprise vertical projections have different cross section in different zones of the flow path, different spacing between said projections, different chemical or biochemical treatment of said projections, a difference in the level of said flow paths, such as forming steps or thresholds between different sections of the flow path etc.

The direction of flow, e.g. the prevention of undesired back flow of sample is chosen among suitable design of the flow paths, the cross section of the substantially vertical projections, an external influence chosen among heating, cooling, irradiation with visible light, infra red irradiation, vibration, and the application of an electric current, or a combination thereof, acting on at least part of said flow paths.

The present invention makes available a chemical or biochemical assay involving a reaction between an analyte in a sample and one or more reagents, wherein the sample is added to a device as defined above. The reaction between said analyte and one or more reagent may be any conventional reaction, presently performed on a solid substrate or in a carrier material. This also comprises assays where a device according to the invention is used for the pre-treatment of the sample.

The present invention also makes available chemical or biochemical assay involving a reaction between an analyte in a sample and one or more reagents, wherein said reaction itself and/or the reading of the result is performed on a device as defined above.

The invention also makes available a method for handling liquid samples, wherein a device as defined above is used.

The device according to the invention is advantageously used in analytical applications where the liquid sample contains particulate matter, such as cells, tissue debris, organic or inorganic matter, other contamination etc, which is desired to separate from the bulk of the sample. One important application is when the liquid sample is whole blood and in such cases, the lateral capillary flow involves the separation of red blood cells from plasma without significant rupture of said cells. According to one embodiment, such separation in general, and in particular the gentle separation of red blood cells, is achieved in a gradient of projections wherein the spacing decreases from about 7 µm to about 1 µm over the length of said filtering zone.

According to one embodiment said receiving zone forms a basin for components separated from the lateral flow, e.g. particulate matter or cells prevented from passing between the projections, or entering that space only to a limited degree.

According to another embodiment the particulate matter travels with the lateral flow. In applications where said liquid sample is whole blood, it is important that said lateral capillary flow involves the transportation of red blood cells without significant rupture of said cells. This is achieved by the present invention through the control of one or more of the parameters of the projections, such as the height, diameter and reciprocal spacing, as well as the chemical or biochemical derivatisation of the projections.

The spacing between said projections can be varied depending on the intended use and the properties of the liquid sample, as well as the properties of components to be separated or transported, and preferably is in the interval of 1 to 100 µm, more preferably in the interval of 1 to 50 µm. The distance between said projections can be chosen by a skilled person, considering which sample the device is intended for, the properties of said sample, and the properties of the components that are to be separated.

The device according to the present invention is built on a plastic substrate, preferably thermoplastic, or a substrate having a plastic upper layer. This can in turn be coated or derivatised, e.g. using techniques such as sputtering, vapour deposition and the like, and given a coating of silicon, a metal or other. The present invention can also be made of silicon substrates. According to a preferred embodiment the substrate is given a hydrophilic treatment or coating, e.g. by subjecting the substrate to an oxidative treatment, such as e.g. gas plasma treatment, coating with a hydrophilic substance such as silicon oxide, hydrophilic polymers such as dextran, polyethylene glycol, heparin and derivatives thereof, detergents, biologic substances such as polymers, etc.

Consequently, according to one embodiment of the invention, said projections or at least a sub-set thereof are provided with a chemical, biologic or physical functionality. The projections may have chemically reactive groups on their surface. The projections may also have substances with biological affinity bound to their surface.

According to another embodiment, the projections carry structures or groups chosen among hydrophilic groups, hydrophobic groups, positively and/or negatively charged groups, silicon oxide, carbohydrates, amino acids, nucleic acids, and macromolecules, or combinations thereof.

According to yet another embodiment, the projections have a physical property selected from the projection diameter, height, reciprocal spacing, shape, cross section, surface coating, the number of projections per unit area, wetting behavior of the surface of said projections, or a combination thereof, according to the desired end use of the substrate.

According to another embodiment, particles are provided chemically or physically bound to the substrate, or mechanically trapped within a region comprising a plurality of projections. Said particles are chosen among commercially available particles, so called beads, and may have a core of glass, metal or polymer, or a combination of these, and they optionally carry on their surface chemical or biological moieties, such as polyclonal antibodies, monoclonal antibodies, amino acids, nucleic acids, carbohydrates or combinations thereof.

The present invention also makes available a device suitable for use in or together with a device for detection of an analyte in a liquid sample, wherein said device has projections substantially vertical to its surface, said projections having a height, diameter and reciprocal spacing such, that said device is capable of separating components of said liquid sample while achieving a lateral flow of said liquid sample. This device may have one or more of the properties and functionalities described above, depending on its intended use.

This device may be used separately, in association with, or integrated in a device for the analysis of a liquid sample. This device may function as a pre-treatment step in or before a conventional analysis.

The present invention also makes available a method for performing an assay on a liquid sample, said sample being applied to a substrate having a zone for receiving the sample, which is in fluid connection with a reaction zone, and optionally a transport or incubation zone connecting the receiving and reaction zone, respectively, wherein said substrate is a non-porous substrate, and said receiving zone, reaction zone and optional transport or incubation zone consist of areas of projections substantially vertical to said surface, and having a height, diameter, and reciprocal spacing such, that lateral capillary flow of said liquid sample in said zone is achieved.

According to one embodiment of this method, a filtering step is performed following the addition of the sample, said filtering effected in a filtering zone by projections substantially vertical to the surface of said substrate, the projections having a height, diameter, and reciprocal spacing forming a gradient with regard to the diameter, and/or reciprocal spacing such that components of the sample are gradually retained.

This method can be used for all applications where components of a liquid sample need to be separated from the bulk of the sample. The method is however particularly suitable for applications where said liquid sample is whole blood and said lateral capillary flow involves the separation of red blood cells from plasma without significant rupture of said cells.

One way to achieve a gentle separation of components of the sample, is to subject the sample to a filtering zone where the spacing between the projections gradually decreases. In applications where the lateral capillary flow involves the separation of red blood cells from plasma, it is important that this takes place without significant rupture of said red blood cells. Ito achieve this, in applications involving the separation of red blood cells, the spacing preferably decreases from about 7 µm to about 1 µm over the length of said filtering zone.

According to one embodiment of the method, components separated from the lateral flow are retained in a basin, substantially prevented from entering the filtering zone.

Another embodiment, in applications where said liquid sample is whole blood, is a method of achieving a lateral capillary flow involving the transportation of red blood cells without significant rupture of said cells. And if needed a flow of buffer liquid can be used to reduce the amount of cells in the reaction zone, promoting the detection step.

The invention also makes available a method for performing an assay on a liquid sample, in particular a sample of whole blood, wherein said sample is added to a device as described above.

The device according to the present invention surprisingly replaces prior art devices where the substrate, and/or one or more of said zones were made of a porous material such as nitrocellulose, cellulose, asbestos fibres, glass fibres and the like.

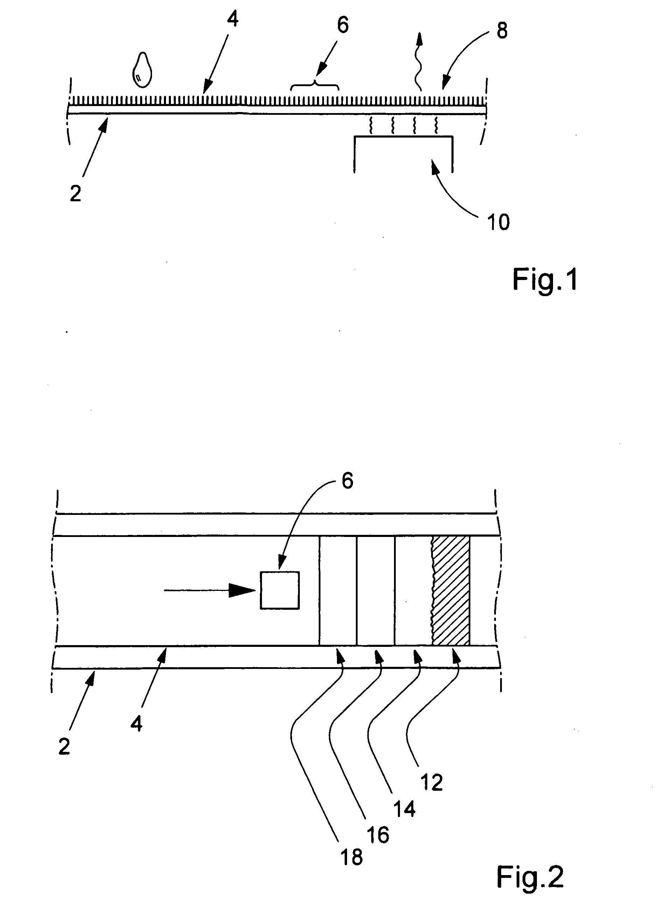

A general embodiment is schematically shown in Fig. 1, showing a part of a device where the surface of a substrate 2 has a flow path 4 comprising projections substantially vertical to said surface, and having a height, diameter and reciprocal spacing such, that lateral capillary flow is achieved. A reaction zone 6 is provided on said surface, and it is desired that sample and optionally reagents, buffers is/are transported laterally so that they pass said reaction zone. It is further desired that the rate, at which said reagents etc pass the reaction zone is controlled. To this end, a sink 8 is provided at the distal end of the substrate, in relation to the place where sample is added. This sink is then heated by heating means 10, in order to evaporate liquid and control or increase the flow rate of the sample.

Another embodiment is illustrated in Fig. 2, showing a part of a substrate 2 having a flow path 4 and a reaction zone 6. The sink is however divided into zones 12, 14, 16, and 18, which can be heated consecutively, starting from the most distal zone 12.

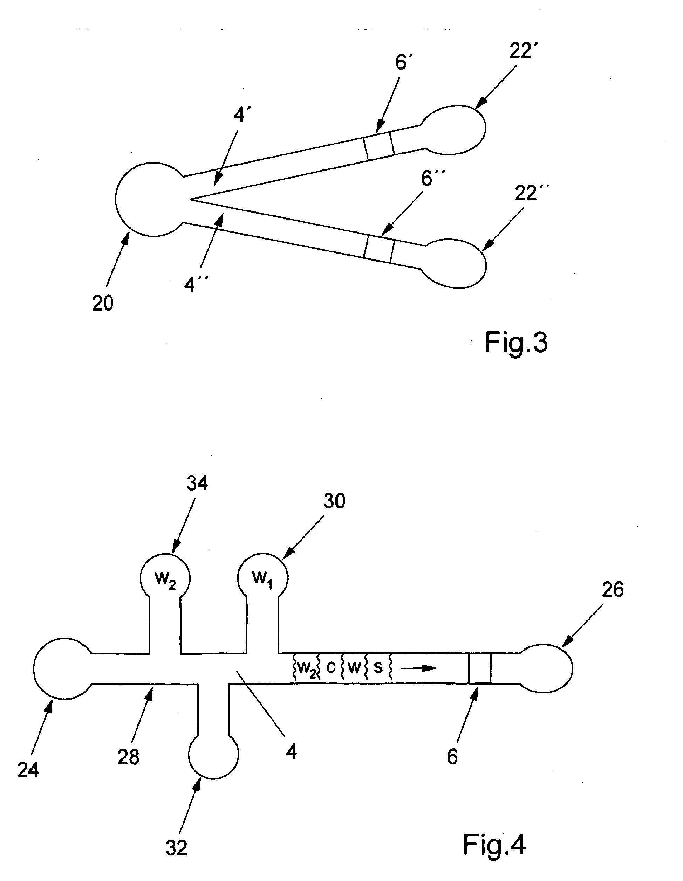

Yet another embodiment is illustrated in Fig. 3, where sample is added to one location 20 in fluid connection with two flow paths 4' and 4", each flow path passing a reaction zone 6'and 6", and ending in a sink, 22' and 22", respectively. By giving the sinks 22' and 22" different capacity, or by subjecting them to heating, it becomes possible to achieve different flow rates in the flow paths 4 and 4". This is advantageous in applications where two or more determinations are to be performed on the same sample, and each determination has its own requirements as to incubation time, reaction time, flow rate etc. While Fig. 3 only shows two flow paths, it is understood that three, four or several parallel flow paths could be provided.

Fig. 4 illustrates an embodiment where sample is added to one location 24, in a system 28 forming a fluid connection between sample addition 24, a flow path 4, a reaction zone 6 and a sink 26, where a first wash buffer W₁ stored in or added to another location 30, a conjugate C stored in or added to 32, and a second wash buffer W₂ stored in or added to 34, can be serially introduced onto said flow path 4. When W₁, W₂, and C are added simultaneously, the distance between 30, 32, and 34; and the flow path 4, respectively, can be varied in order to influence the time it takes W₁, W₂, and C to reach the flow path. When W₁, W₂, and C are stored on the surface, at the locations 30, 32, and 34, respectively, the release of these components can be controlled by the provision of meltable seals, the heating or cooling of the components, or other means, influencing the time it takes W₁, W₂, and C to reach the flow path. While 30, 32, and 34 are schematically shown in Fig. 4 to be at an approximately equal distance from the flow path 4, and approximately vertical thereto, it is contemplated that they can be at different distances, at an angle to the flow path, or even partially parallel thereto. Centrifugation is contemplated as one means to deliver one or all of W₁, W₂, and C to the flow path.

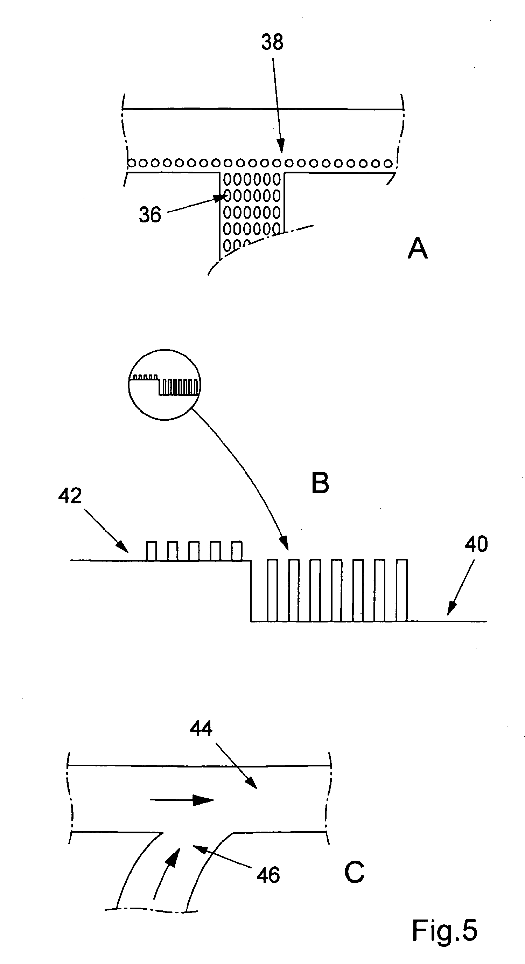

Fig. 5A schematically illustrates an embodiment where the transition from one flow path 36 into another low path 38 is characterised in that the substantially vertical projections in said flow paths have different properties, said properties chosen so that the direction of flow is controlled, e.g. creating a preferred direction of flow. Different properties in this respect can be different cross section, height, orientation, spacing, and surface chemistry of the projections, or a combination thereof.

Fig. 5B schematically illustrates another embodiment where not only the properties of said projections, but also the level of the flow paths are different, again in order to create a preferred direction of flow.

Finally, Fig. 5C schematically illustrates an embodiment where the geometry of the respective flow paths 44 and 46 has been designed to create a preferred direction of flow.

The above embodiments may also be combined, e.g. by taking features from one embodiment and introducing these in another.

### Advantages of the invention

An advantage of the device according to the invention is the possibility to accurately control the flow rate, including possibilities to stop and start the flow. This in turn makes it possible to perform simultaneous or sequential reactions, either in series or in parallel, in a controlled fashion. It is also possible to influence the incubation time of different reactions.

Another advantage of the device is that, due to the open, regular structure and the defined properties of the capillary flow zones, the addition of reagents these zones or the derivatisation of the surface of the projections is greatly simplified.

Yet another advantage of the device is the uniformity of the structure not only within a single device, but also between all devices produced. This result in significantly increased reliability and repeatability of the assays built on the inventive device.

An important advantage of the inventive device is that the degree of separation, from none to total, of the blood cells, can be accurately controlled.

The inventive device has many advantages with respect to the manufacturing process. All capillary zones can be made in one step and no assembly of parts is required. Optionally, a cover having at least one aperture for sample addition and one reading the result of the assay can be placed over the substrate and the capillary zones.

Although the invention has been described with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto.

## Claims

1. A device for handling liquid samples, said device comprising at least one flow path (4) and at least one zone for (20, 24) receiving the sample, and a transport or incubation zone, **characterized in that** said device further comprises a sink (8) with a capacity of receiving said liquid sample and supporting or controlling the flow rate of said sample through said transport or incubation zone, said sink (8) comprising an area having projections substantially vertical to its surface, and said sink (8) being adapted to respond to external influence regulating its capacity to receive said liquid sample.

2. The device according to claim 1, wherein two or more flow paths are provided, each connected to one sink respectively, said device adapted for performing multiple analyses on one sample.

3. The device according to claim 1, wherein two or more flow paths are provided, each connected to one and the same sink, said device adapted for performing multiple analyses on one sample.

4. The device according to one of claims 2 - 3, wherein said multiple analyses are performed in parallel.

5. The device according to claim 1, wherein multiple reagents, buffers, etc can be serially added to a flow path.

6. The device according to any one of claims 1 - 5, wherein the external influence regulating the capacity of said sink to receive said liquid sample is chosen among heating, cooling, irradiation with visible light, infra red irradiation, vibration, and the application of an electric current.

7. The device according to claim 6, wherein the sink can be divided into subsections, suitable for being serially subjected to said external influence.

8. The device according to claim 6 or 7, wherein the sink or a sub-section thereof is heated to evaporate liquid sample there from.

9. The device according to any one of claims 1 - 8, wherein one or more flow paths in fluid connection with the sink are chosen among flow paths formed as a capillary groove or open channel, a closed capillary, a tortuous path through a fibrous or through a gel-like material.

10. The device according to any one of claims 1 - 8, wherein one or more flow paths in fluid connection with the sink comprise areas having substantially vertical projections.

11. The device according to claim 10, wherein said vertical projections have different cross section in different zones of the flow path.

12. The device according to claim 1, wherein back flow of sample is prevented by suitable design of the at least one flow path, the cross section of the substantially vertical projections, an external influence chosen among heating, cooling, irradiation with visible light, infra red irradiation, vibration, and the application of an electric current, or a combination thereof, acting on at least part of said flow paths.

13. A chemical or biochemical assay involving a reaction between an analyte in a sample and one or more reagents, **characterized in that** the sample is added to a device according to any one of claims 1 - 12.

14. A chemical or biochemical assay involving a reaction between an analyte in a sample and one or more reagents, **characterized in that** said reaction is performed on a device according to any one of claims 1 - 12.

15. A method for handling liquid samples, **characterized in that** a device according to any one of claims 1 - 12 is used.

## Patentansprüche

1. Eine Vorrichtung zur Behandlung flüssiger Proben, umfassend zumindest einen Fließweg (4) und zumindest eine Zone (20, 24) zum Aufnehmen der Probe und eine Transport- oder Inkubationszone, **dadurch gekennzeichnet, dass** die Vorrichtung weiter eine Auffangzone (8) umfasst, mit einer Kapazität zum Aufnehmen der flüssigen Probe und zum Unterstützen oder Kontrollieren der Fließgeschwindigkeit der Probe durch die Transport- oder Inkubationszone, wobei die Auffangzone (8) einen Bereich umfasst, der Vorsprünge im Wesentlichen senkrecht zu seiner Oberfläche besitzt, und wobei die Auffangzone (8) angepasst ist, auf einen externen Einfluss zu reagieren, der ihre Kapazität zum Aufnehmen der Probe reguliert.

2. Vorrichtung nach Anspruch 1, wobei zwei oder mehr Fließwege vorgesehen sind, die jeder mit jeweils einer Auffangzone verbunden sind, wobei die Vorrichtung angepasst ist, mehrere Analysen an einer Probe durchzuführen.

3. Vorrichtung nach Anspruch 1, wobei zwei oder mehr Fließwege vorgesehen sind, die jeder mit ein und derselben Auffangzone verbunden sind, wobei die Vorrichtung angepasst ist, mehrere Analysen an einer Probe durchzuführen.

4. Vorrichtung nach einem der Ansprüche 2 - 3, wobei mehrere Analysen parallel durchgeführt werden.

5. Vorrichtung nach Anspruch 1, wobei mehrere Reagenzien, Puffer, etc. hintereinander einem Fließweg zugegeben werden können.

6. Vorrichtung nach einem der Ansprüche 1 - 5, wobei der externe Einfluss, der die Kapazität der Auffangzone zum Aufnehmen der flüssigen Probe reguliert, ausgewählt ist aus Erwärmung, Kühlung, Bestrahlung mit sichtbarem Licht, Infrarotbestrahlung, Vibration und dem Anlegen eines elektrischen Stroms.

7. Vorrichtung nach Anspruch 6, wobei die Auffangzone in Teilbereiche aufgeteilt sein kann, die geeignet sind, hintereinander dem externen Einfluss unterworfen zu werden.

8. Vorrichtung nach Anspruch 6 oder 7, wobei die Auffangzone oder ein Teilbereich derselben erwärmt wird, um flüssige Probe daraus zu verdampfen.

9. Vorrichtung nach einem der Ansprüche 1 - 8, wobei ein oder mehrere Fließwege, die in flüssiger Verbindung mit der Auffangzone stehen, ausgewählt sind aus Fließwegen, die als eine kapillare Fuge oder als offener Kanal, als eine geschlossene Kapillare, als ein gewundener Weg durch ein fasriges oder durch ein gelartiges Material ausgebildet sind.

10. Vorrichtung nach einem der Ansprüche 1 - 8, wobei ein oder mehrere Fließwege, die in flüssiger Verbindung mit der Auffangzone stehen, Bereiche umfassen, die im Wesentlichen senkrechte Vorsprünge besitzen.

11. Vorrichtung nach Anspruch 10, wobei die senkrechten Vorsprünge unterschiedlichen Querschnitt in unterschiedlichen Zonen des Fließwegs haben.

12. Vorrichtung nach Anspruch 1, wobei ein Zurückfließen der Probe verhindert wird durch eine geeignete Ausgestaltung des zumindest einen Fließwegs, des Querschnitts der im Wesentlichen senkrechten Vorsprünge, eines auf zumindest einen Teil der Fließwege einwirkenden externen Einflusses ausgewählt aus Erwärmung, Kühlung, Bestrahlung mit sichtbarem Licht, Infrarotbestrahlung, Vibration und dem Anlegen eines elektrischen Stroms, oder einer Kombination daraus.

13. Ein chemischer oder biochemischer Test, der eine Reaktion zwischen einem Analyten in einer Probe und einem oder mehreren Reagenzien involviert, **dadurch gekennzeichnet, dass** die Probe einer Vorrichtung nach einem der Ansprüche 1 - 12 zugegeben wird.

14. Ein chemischer oder biochemischer Test, der eine Reaktion zwischen einem Analyten in einer Probe und einem oder mehreren Reagenzien involviert, **dadurch gekennzeichnet, dass** die Reaktion in einer Vorrichtung nach einem der Ansprüche 1 - 12 durchgeführt wird.

15. Ein Verfahren zum Behandeln flüssiger Proben, **dadurch gekennzeichnet, dass** eine Vorrichtung nach einem der Ansprüche 1 - 12 benutzt wird.

## Revendications

1. Dispositif servant à manipuler des échantillons liquides, lequel dispositif comprend au moins un trajet d'écoulement (4) et au moins une zone (20, 24) de réception d'échantillon, ainsi qu'une zone de transport ou d'incubation, **caractérisé en ce que** ce dispositif comprend en outre un puits (8) capable de recevoir ledit échantillon liquide et de soutenir ou de réguler le débit dudit échantillon dans ladite zone de transport ou d'incubation, lequel puits (8) comporte une zone dont la surface porte des saillies sensiblement verticales, et lequel puits (8) est adapté pour répondre à une influence extérieure régulant sa capacité à recevoir ledit échantillon liquide.

2. Dispositif conforme à la revendication 1, dans lequel il y a deux trajets d'écoulement ou plus, chacun en relation avec son puits, et lequel dispositif est adapté à la réalisation de multiples analyses sur un seul échantillon.

3. Dispositif conforme à la revendication 1, dans lequel il y a deux trajets d'écoulement ou plus, tous en relation avec un seul et même puits, et lequel dispositif est adapté à la réalisation de multiples analyses sur un seul échantillon.

4. Dispositif conforme à l'une des revendications 2 et 3, dans lequel lesdites analyses multiples sont réalisées en parallèle.

5. Dispositif conforme à la revendication 1, dans lequel on ajoute en série de multiples réactifs, agents tampons, etc., sur un trajet d'écoulement.

6. Dispositif conforme à l'une des revendications 1 à 5, dans lequel l'influence extérieure qui régule la capacité dudit puits à recevoir ledit échantillon liquide est choisie parmi un chauffage, une réfrigération, une irradiation avec de la lumière visible ou avec un rayonnement infra-rouge, des vibrations, et le passage d'un courant électrique.

7. Dispositif conforme à la revendication 6, dans lequel le puits peut être divisé en subdivisions, adaptées pour être soumises en série à ladite influence extérieure.

8. Dispositif conforme à la revendication 6 ou 7, dans lequel on chauffe le puits ou l'une de ses subdivisions pour faire s'en évaporer un échantillon liquide.

9. Dispositif conforme à l'une des revendications 1 à 8, dans lequel un ou plusieurs trajets d'écoulement en relation de fluide avec le puits sont choisis parmi des trajets d'écoulement ayant l'aspect d'une rainure ou d'un chenal ouvert capillaire, d'un capillaire fermé, ou d'un trajet sinueux au travers d'un matériau fibreux ou d'un matériau de type gel.

10. Dispositif conforme à l'une des revendications 1 à 8, dans lequel un ou plusieurs trajets d'écoulement en relation de fluide avec le puits comportent des zones qui portent des saillies sensiblement verticales.

11. Dispositif conforme à la revendication 10, dans lequel lesdites saillies verticales présentent, dans différentes zones du trajet d'écoulement, différentes sections transversales.

12. Dispositif conforme à la revendication 1, dans lequel on empêche l'échantillon de s'écouler à rebours, grâce à une conception appropriée dudit ou desdits trajets d'écoulement, à la section transversale des saillies sensiblement verticales, ou à une influence extérieure choisie parmi un chauffage, une réfrigération, une irradiation avec de la lumière visible ou avec un rayonnement infra-rouge, des vibrations, et le passage d'un courant électrique, ou une combinaison de telles influences, agissant sur au moins une partie dudit ou desdits trajets d'écoulement.

13. Test chimique ou biochimique impliquant une réaction entre un analyte présent dans un échantillon et un ou plusieurs réactifs, **caractérisé en ce qu'**on amène l'échantillon dans un dispositif conforme à l'une des revendications 1 à 12.

14. Test chimique ou biochimique impliquant une réaction entre un analyte présent dans un échantillon et un ou plusieurs réactifs, **caractérisé en ce que** cette réaction est effectuée sur un dispositif conforme à l'une des revendications 1 à 12.

15. Procédé de manipulation d'échantillons liquides, **caractérisé en ce qu'**on se sert d'un dispositif conforme à l'une des revendications 1 à 12.
